# EUROPEAN PATENT APPLICATION

(11) **EP 4 548 868 A1**
(43) Date of publication of application: **07.05.2025**
(21) Application number: 23383112.2
(22) Date of filing: 31.10.2023
(51) Int. Cl.: A61B 18/00

(54) **CATHETER TIP HAVING REFLECTIVE SURFACE AND INTEGRATED IRRIGATION CHANNELS**

(71) Applicant: Medlumics S.L., 28760 Tres Cantos, Madrid (ES)
(72) Inventor: Duperron, Matthieu, 28045 Madrid (ES); Sancho, Juan, 46650 Canals, Valencia (ES); Roigé, Abel, 28100 Alcobendas (ES); Herranz, David, 28430 Alpedrete (ES); Mas, Sara, 28760 Tres Cantos (ES); Pantoja, Jacobo, 28050 Madrid (ES); Galiano, Vanessa, 28025 Madrid (ES); Maroñas, Patricia, 28770 Colmenar Viejo (ES); González, Alejandro, 28770 Colmenar Viejo (ES); Romoscanu, Alexander, 1270 Geneve (CH); Jimenez, Jorge, Atlanta, GA (US); Bailleul, Christophe, 75011 Paris (FR); Greene, James, Shelfield Green Warwickshire, B496JR (GB)
(74) Representative: DTS Patent- und Rechtsanwälte PartmbB

(57) **Abstract**

A catheter tip to be disposed at the distal end of an ablation catheter may include a proximal end for connection to the ablation catheter shaft and a distal end containing an ablation electrode. The distal end comprises a reflector element having a body or optically transparent material, one or more irrigation ports, and a plurality of optical ports. The reflector element comprises one or more irrigation longitudinally-extending channelsconfigured to be fluidically coupled to the irrigation ports. The reflector element further includes a reflective surface configured to reflect light along a pathway between an axial direction and a radial direction. In some embodiments, the reflective surface includes a plurality of reflective facets disposed around an axially aligned opening, the reflective facets separated by non-reflective surfaces.

## Description

### BACKGROUND

### Field

Embodiments of the present disclosure relate to a catheter tip, in particular to a catheter tip transmitting both illumination and a fluid.

### Background

Ablation is a medical technique for producing tissue necrosis. It is used to help treat different pathologies including cancer, Barret's esophagus, renal denervation, pulmonary denervation or cardiac arrhythmias, among others. Various energy sources may be utilized for ablation. For example, in radiofrequency (RF) ablation, an external electrode is placed on a patient's body, and an alternating potential is applied to the tip of a catheter that is placed in contact with the tissue to be treated within the patient's body. The application of alternating current with an oscillating frequency above several hundreds of kHz avoids the stimulation of excitable tissue while delivering heat by means of the Joule's effect. The increase in tissue temperature produces denaturation of the biological molecules, including proteins such as collagen, myosin, or elastin.

### BRIEF DESCRIPTION OF THE DRAWINGS/FIGURES

The accompanying drawings, which are incorporated herein and form a part of the specification, illustrate embodiments of the present disclosure and, together with the description, further serve to explain the principles of the disclosure and to enable a person skilled in the pertinent art to make and use the disclosure.
FIG. 1 illustrates an example system for ablation, according to embodiments of the present disclosure.
FIG. 2 illustrates an example ablation catheter, according to embodiments of the present disclosure.
FIGs. 3A-3K illustrate an example catheter tip, according to embodiments of the present disclosure.
FIGs. 4a-d illustrate a preferred embodiment of a reflector element of the present disclosure.
FIG. 5 illustrates an example catheter tip incorporating the reflector element of FIG. 4a-d.

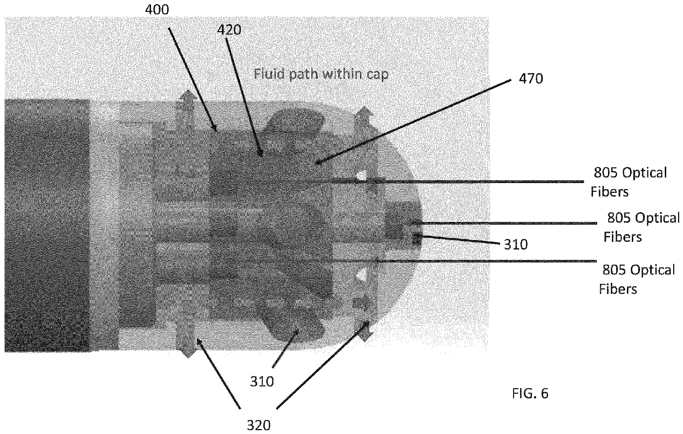

FIG. 6 illustrates an example of fluid movement through a catheter tip of FIG. 5.

Embodiments of the present disclosure will be described with reference to the accompanying drawings.

### DETAILED DESCRIPTION

Although specific configurations and arrangements are discussed, it should be understood that this is done for illustrative purposes only. A person skilled in the pertinent art will recognize that other configurations and arrangements can be used without departing from the spirit and scope of the present disclosure. It will be apparent to a person skilled in the pertinent art that this disclosure can also be employed in a variety of other applications.

It is noted that references in the specification to "one embodiment," "an embodiment," "an example embodiment," etc., indicate that the embodiment described may include a particular feature, structure, or characteristic, but every embodiment may not necessarily include the particular feature, structure, or characteristic. Moreover, such phrases do not necessarily refer to the same embodiment. Further, when a particular feature, structure or characteristic is described in connection with an embodiment, it would be within the knowledge of one skilled in the art to effect such feature, structure or characteristic in connection with other embodiments whether or not explicitly described.

It should be noted that although this application may refer specifically to cardiac ablation, the embodiments described herein may target other pathologies as well, along with additional energy sources for ablation, including but not limited to radiofrequency (RF), microwave, laser, ultrasound, and pulsed field ablation (PFA). The principles of using laser energy to treat other pathologies are similar, and therefore the techniques used to apply the laser energy are similar.

Disclosed herein are embodiments of an ablation catheter capable of both optical tissue analysis and ablation, in which the ablation catheter tip includes an optical structure for directing light for evaluating target tissue. In some embodiments, the optical structure of the catheter may be configured to transmit beams of exposure radiation to a sample and receive one or more beams of scattered radiation that have been reflected or scattered from the sample in order to optically interrogate the sample. Optical interrogation techniques may include, for example, low-coherence interferometry (LCI), optical coherence tomography (OCT), optical coherence refractometry (OCR), or other optical modalities to perform imaging, obtain optical measurement data, identify optical properties of the target tissue, etc. Optical properties may include, for example and without limitation, polarization and/or birefringence. Birefringence, or a loss of birefringence, may be correlated with necrosis and muscle fiber denaturation. Optical properties may include spectral information, and/or other properties that would be appreciated by a person of ordinary skill in the art.

In addition to the optical structure used to direct light for interrogation, an ablation catheter according to embodiments described herein may also include one or more ablation electrodes. Ablation involves application of sufficient energy to cause death of the target tissue cells. During ablation, excess heat may build in up in the catheter tip. While irrigation fluid can be used to cool the catheter tip, existing optical interrogation systems interrupt the flow of irrigation fluid and prevent such fluid from reaching all parts of the heated tip.

Accordingly, embodiments of the present disclosure provide a reflector element located within the catheter tip that directs optical interrogation light to tissue around the catheter tip, while allowing irrigation fluid to pass through one or more irrigation channels in the optical structure to the distal end of the ablation tip for cooling.

Herein, the terms "electromagnetic radiation," "light," and "beam of radiation" are all used to describe the same electromagnetic signals propagating through the various described elements and systems.

### Example Catheter System and Console Embodiments

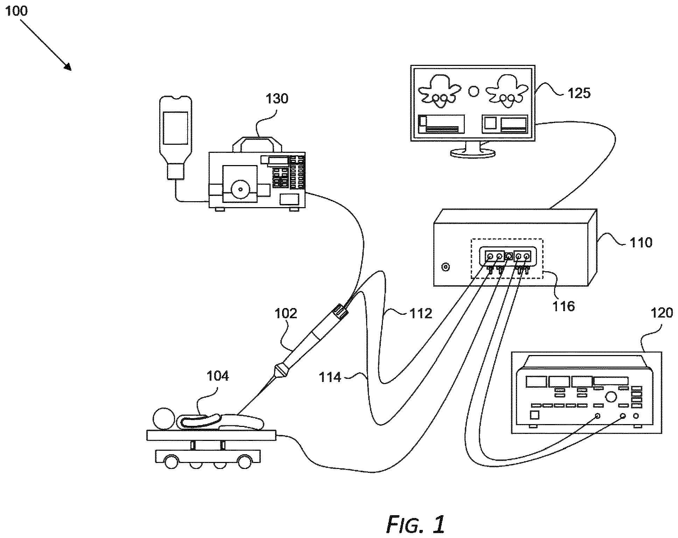

In some embodiments, an ablation catheter and console system described herein uses optical coherence tomography (OCT) and/or optical coherence reflectometry (OCR), refractometry, or other methods to perform tissue ablations, track scar formation in realtime, and monitor/verify lesion geometries and isolation by directly observing the scar pattern in tissue. FIG. 1 illustrates a diagram of an example system 100 for performing ablation according to embodiments of the present disclosure. The system 100 includes catheter 102, console 110, signal generator 120, display 125, and irrigation pump 130. The catheter 102, console 110, signal generator 120, display 125, and irrigation pump 130 may be communicatively coupled together via wired and/or wireless connections. In some aspects, console 110 may receive electrical and/or magnetic field information from a plurality of field generators or sensors such as the patches 902 illustrated in FIG. 9, described above. In some aspects, catheter 102 may represent an exemplary aspect of catheter 200 as described below. In some aspects, a distal section of catheter 102 is positioned at a portion of tissue in patient 104. It is understood that the aspects described herein may be used in vivo and/or in vitro.

In some aspects, catheter 102 may be positioned at a portion of tissue subject to ablation using energy generated by signal generator 120. In some aspects, signal generator 120 may be an electronic device configured to generate radiofrequency (RF), cryogenic, or electroporation (e.g., pulsed electric field (PFA)) signals for ablation. The signal generator 120 may be coupled to catheter 102 directly or via the console 110 and may send energy to catheter 102 to ablate a portion of tissue at a selected tissue site. In some aspects, the portion of tissue may include myocardial tissue, cardiac muscle tissue, skeletal tissue, or the like. Energy may be applied to the portion of tissue through optical view ports in the distal section of catheter 102. After applying the energy, structural changes in the tissue may be observed by acquiring optical signals via one or more optical viewports of catheter 102.

Console 110 may comprise a computing device configured to acquire the optical signals from catheter 102 and analyze the optical signals to detect changes in optical properties of the tissue. In some aspects, console 110 may include hardware (e.g., circuits), firmware, software, or any combination thereof to perform analysis of the optical signals and generate a birefringence map as described herein. In some aspects, console 110 may send light through an optical circuit within itself and the catheter 102 and into the tissue to monitor scar progression, contact between the tissue and catheter 102, and other characteristics of the tissue. In some aspects, console 110 may be referred to herein as a control console, a processing device, and/or a controller. Console 110 may be coupled to display 125, which may present results from the optical signal analysis and lesion predictions and allow a user to select/view, modify, and/or control parameters related to the operation of catheter 102, console 110, signal generator 120, and/or irrigation pump 130.

In some aspects, irrigation pump 130 may be coupled to catheter 102 via a tubing. In some aspects, irrigation pump 130 may allow for fluid to be pumped through the tubing and released at the tissue site through catheter 102 (e.g., through optical view ports or through separate irrigation slits at the distal section of catheter 102). Fluid from the irrigation pump 130 may cool the distal section of catheter 102 and the surrounding tissue during ablation, and also flush away any debris during and/or after ablation.

In some aspects, catheter 102 may be coupled to console 110 via one or more optical connections 112 and one or more electrical connections 114. Optical connections 112 may include single mode optical fibers and/or multimode optical fibers that allow acquisition and/or transmission of optical signals to and from catheter 102 and console 110 for further analysis. Electrical connections 114 may include wiring, pins, and/or components used for supplying power and energy from signal generator 120 to catheter 102 for ablation.

In some aspects, the optical and electrical connections 112, 114 may be connected to console 110 via a communication interface 116. Communication interface 116 may allow for transmission of various signals (e.g., optical and electrical signals) between catheter 102 and console 110. In some aspects, the communication interface 116 may include a connector that facilitates proper alignment of optical fibers between the catheter 102 and console 110. In some aspects, the connector design may include both electrical and optical extension lines.

### Exemplary Catheter Embodiments

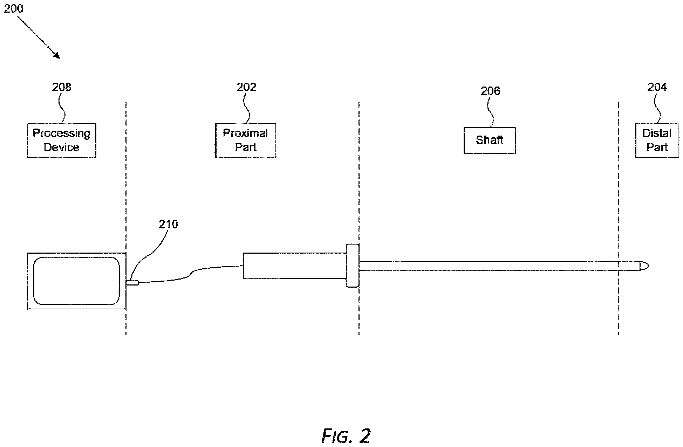

FIG. 2 illustrates a catheter 200 according to aspects of the present disclosure. Catheter 200 includes a proximal section 202, a distal section 204, and a shaft 206 coupled between proximal section 202 and distal section 204. In an aspect, shaft 206 includes one or more radiopaque markers for navigation purposes. In one aspect, catheter 200 includes a communication interface 210 between catheter 200 and a processing device 208. Communication interface 210 may include one or more optical fibers and connectors between processing device 208 and catheter 200. In other examples, communication interface 210 may include an interface component that allows wireless communication, such as Bluetooth, Wi-Fi, cellular, and the like, to communicate with the catheter 200 or other processing components in a catheter system.

Proximal section 202 may house various electrical and optical components used in the operation of catheter 200. In some embodiments, an optical source may be included within proximal section 202 to generate a source beam of radiation for optical evaluation. In some embodiment, the optical source can be an external optical source coupled to the proximal section via optical connections 112 in FIG. 1. The optical source may include one or more lasers, laser diodes, light emitting diodes (LEDs), or other kinds of optical coherent source. The beam of radiation generated by the optical source may have a wavelength within the infrared range. In one example, the beam of radiation has a central wavelength between about 1000 nm and 1600 nm. The optical source may be designed to output a beam of radiation at only a single wavelength, or it may be a swept source and be designed to output a range of different wavelengths. The generated beam of radiation may be guided towards distal section 204 via the optical transmission medium connected between proximal section 202 and distal section 204 within shaft 206. Some examples of optical transmission media include single-mode optical fibers and/or multimode optical fibers. In one embodiment, the electrical transmission medium and the optical transmission medium are provided by the same hybrid medium allowing for both electrical and optical signal propagation.

In some aspects, proximal section 202 may include a second optical source, such as a laser energy source, to generate laser energy that is applied at distal section 204 for tissue ablation. In some aspects, proximal section 202 may include a non-optical energy source for generating non-optical energy, such as RF energy, to be applied at distal section 204 for tissue ablation. In some aspects, proximal section 202 may include both a second optical and a non-optical energy source for the selection of energy to be applied at distal section 204. In some aspects, processing device 208 may include one or more components, such as detectors, electronics, and/or other components of an optical circuit/system as described herein. In other aspects, one or more components, such as detectors, electronics, and/or other components of an optical circuit/system may be included in the proximal section 202.

In an embodiment, proximal section 202 includes one or more components of an interferometer in order to perform coherence interferometry using the light generated from the optical sources. Due to the nature of interferometric data analysis, in an embodiment, the optical transmission medium used for guiding the light to and from distal section 204 does not affect the state and degree of light polarization. In another embodiment, the optical transmission medium can affect the polarization in a constant and reversible way. In some embodiments, catheter 200 may include an optical circuit with one or more elements configured to conduct optical spectroscopy. In such embodiments, at least part of the optical path may be made up of multi-mode optical transmission media (e.g., multi-mode optical fiber).

Proximal section 202 may include further interface elements with which a user of catheter 200 can control the operation of catheter 200. For example, proximal section 202 may include a deflection control mechanism that controls a deflection angle of distal section 204. The deflection control mechanism may include a mechanical movement of an element on proximal section 202, or the deflection control mechanism may use electrical and/or mechanical connections to control the movement of distal section 204. Proximal section 202 may include various buttons or switches that allow a user to control when the beams of radiation are transmitted from distal section 204, allowing for the acquisition of optical data. In some embodiments, proximal section 202 may include a deflection control mechanism for controlling one or more pull wires that are coupled to the distal section 204. In some embodiments, thee deflection control mechanism and one or more pull wires allow for steering of the distal section of catheter 200 in order to maneuver within and target specific tissue regions for ablation.

Distal section 204 includes a plurality of optical viewports. In some embodiments, the plurality of optical viewports may be referred to herein as orifices in the catheter tip. In an embodiment, one or more of the optical viewports are machined into the outer body of distal section 204. The optical view ports may be distributed over the outside of distal section 104, resulting in a plurality of distinct viewing directions. In some embodiments, the optical view ports may transmit and collect light (e.g., optical signals) at various angles from the distal section 204. In an embodiment, each of the plurality of viewing directions are substantially non-coplanar. In some aspects, the optical viewports may also be designed with irrigation functionality to cool distal section 204 and surrounding tissue during ablation. In some aspects, the optical viewports are filled with a light-transparent material such that fluid and debris may not pass through the optical viewport.

### Exemplary Catheter Tip and Optical Fiber Alignment Embodiments

Disclosed herein are embodiments of an ablation catheter, including components for diverting optical interrogation energy (e.g., laser light emission) while allowing irrigation fluid to pass to the distal end of the catheter tip.

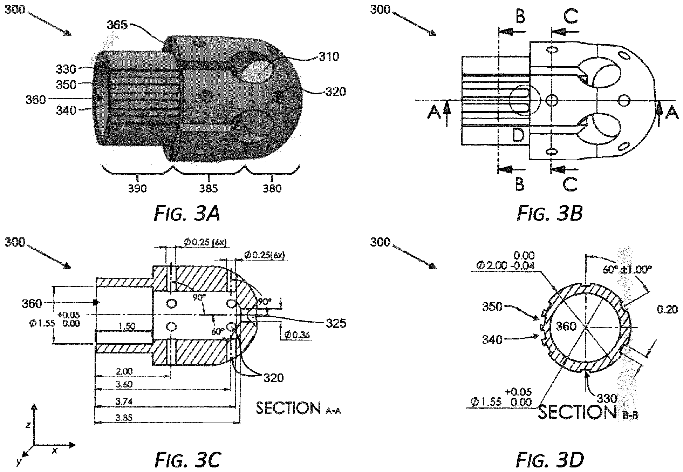

Various views of a catheter tip 300 embodiment are shown in the example diagrams of FIGs. 3A-3K.

FIG. 3A illustrates a diagram of an example catheter tip 300, according to embodiments of the present disclosure. In some embodiments, the catheter tip 300 in FIG. 3A may represent an example embodiment of distal section 104 of the catheter 100 shown in FIG. 1. The catheter tip 300 includes a plurality of optical ports 310, a plurality of irrigation ports 320, a plurality of optical fiber alignment channels 330, an optional RF tube channel 340, an optional thermocouple tube channel 350, and an axial channel 360. In some embodiments, the axial channel 360 is configured to direct an irrigation fluid into the catheter tip 300 and out through the irrigation ports 320.

FIG. 3A further illustrates a side view of the catheter tip 300. In some embodiments, the catheter tip 300 may include a distal end, a body, and a proximal end as illustrated in other catheter tip 300 views described herein. In some embodiments, the catheter tip 300 shown in FIG. 3A can have a monoblock (e.g., unibody) configuration. In some embodiments, the catheter tip 300 can be constructed from a single component or multiple components to facilitate assembly. In some embodiments, the optical fiber alignment channels 330 on the sides of the catheter tip 300 can be used to prevent optical fiber bending, thus, reducing stress on the optical fibers.

FIGs. 3A-3K illustrate diagrams of an example catheter tip 300 with a monoblock configuration, according to embodiments of the present disclosure. In some embodiments, the catheter tip 300 shown in, e.g., FIGs. 3A-3C can be manufactured as a single monoblock component. In some embodiments, FIG. 3A illustrates the catheter tip 300 without any optical fibers or lenses attached for illustrative purposes. The catheter tip 300 can include a distal end 380, a body 385, and a proximal end 390. The distal end 380 may include a plurality of the irrigation ports 320. While only three of the irrigation ports 320 are shown in the distal end 380 for illustrative purposes, it is understood that there may be any number of the irrigation ports 320 in the distal end 602 of the catheter tip 300. For example, similar irrigation ports 320 can be disposed across in a similar manner on the other (non-illustrated) side of distal end 380.

FIGs. 3A-3K also illustrate example optical fiber channels 330, according to embodiments of the present disclosure. The catheter tip 300 includes a plurality of optical fiber channels 330 aligned with a plurality of optical ports 310. In some embodiments, oe or more fibers in a bundle of optical fibers can be inserted into respective optical fiber channels 330. In some embodiments, the distal end 380 and the body 385 can include the plurality of optical ports 310, in which each optical port 310 corresponds to a respective optical fiber (or optical fiber bundle).

FIG. 3A illustrates a three-dimensional view of the catheter tip 300 having a unibody configuration, according to embodiments of the present disclosure. In some embodiments, the catheter tip 300 shown in FIG. 3A may have a plurality of optical ports 310, irrigation ports 320, and a shoulder 365 to accommodate fixing the catheter tip 300 into the catheter shaft 106 (*see* FIG. 1).

FIG. 3B illustrates a side-view diagram of an example catheter tip 300, according to embodiments of the present disclosure. FIG. 3B illustrates a set of cross-section guide lines, identifying cross sections that are detailed further in FIGs. 3C-3E.

FIG. 3C illustrates a diagram of an example of the catheter tip 300, according to some embodiments of the present disclosure. FIG. 3C depicts a cross-sectional view corresponding to the line referred to as A-A in FIG. 3B. Notably depicted in the example of FIG. 3C are the irrigation ports 320 and a distal end optical/irrigation port (e.g., the distal port) 325. Also shown is the axial channel 360. Exemplary dimensions are in millimeters (mm).

FIG. 3D illustrates a diagram of an example of the catheter tip 300, according to some embodiments of the present disclosure. FIG. 3D depicts a cross-sectional view of the catheter tip 300 along the line referred to as B-B in FIG. 3B. Shown in FIG. 3D is the axial channel 360, the optical fiber alignment channels 330, the optional RF tube channel 340, and the optional thermocouple tube channel 350. In some embodiments, the optical fiber alignment channels can be spaced equally radially about the catheter tip 300. In optional embodiments, the optical fiber alignment channels can be radially spaced about the catheter tip 300 in any desired configuration.

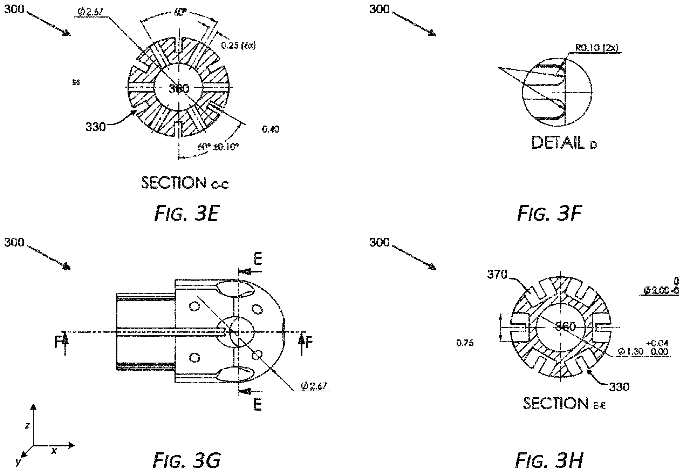

FIG. 3E illustrates a diagram of an example of the catheter tip 300, according to some embodiments of the present disclosure. FIG. 3E depicts a cross-sectional view of the catheter tip 300 along the line referred to as C-C in FIG. 3B. Shown in FIG. 3E is the axial channel 360, the optical fiber alignment channels 330, and the irrigation ports 320.

FIG. 3F illustrates a diagram of an example of the catheter tip 300, according to some embodiments of the present disclosure. FIG. 3F is a detail view of region D shown in FIG. 3B. Region D shows the distal end of the optional RF tube channel 340, and the distal end of the optional thermocouple tube channel 350. Notably shown in FIG. 3F, the distal end of the optional RF tube channel 340, and the distal end of the optional thermocouple tube channel 350 can terminate at an assembly ledge 365 (*see* FIG. 3A) of the catheter tip 300. In some embodiments, the assembly ledge 365 provides a stopping point when the catheter tip 300 is inserted into the catheter shaft 106 shown in FIG 1.

FIG. 3G illustrates a side-view diagram of an example catheter tip 300, according to embodiments of the present disclosure. FIG. 3G illustrates a set of cross-section guide lines, identifying cross sections that are detailed further in FIGs. 3H-3I.

FIG. 3H illustrates a diagram of an example of the catheter tip 300, according to some embodiments of the present disclosure. FIG. 3H depicts a cross-sectional view of the catheter tip 300 along the line referred to as E-E in FIG. 3G. Shown in FIG. 3H is the axial channel 360, the optical fiber alignment channels 330, and a plurality of reflective facets 370. Notably shown in the example of FIG. 3H is that the optical fiber alignment channels 330 are aligned with, and centered upon, the reflective facets 370. In some embodiments, the optical fiber alignment channels and the reflective facets 370 can be spaced equally radially about the catheter tip 300.

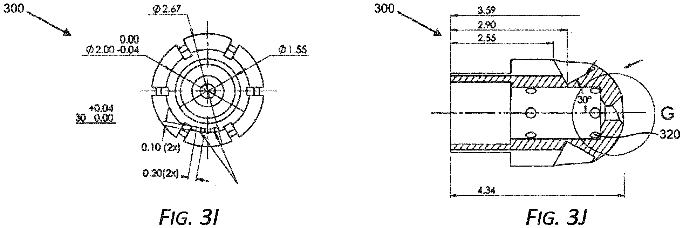

FIG. 3I illustrates a diagram of an example of the catheter tip 300, according to some embodiments of the present disclosure. FIG. 3I depicts a cross-sectional view of the catheter tip 300 at the assembly ledge 365.

FIG. 3J illustrates a diagram of an example of the catheter tip 300, according to some embodiments of the present disclosure. FIG. 3J depicts a cross-sectional view of the catheter tip 300 along the line referred to as F-F in FIG. 3G. Shown in FIG 3J is the axial channel 360, the reflective facets 370, the optical ports 310, and the irrigation ports 320.

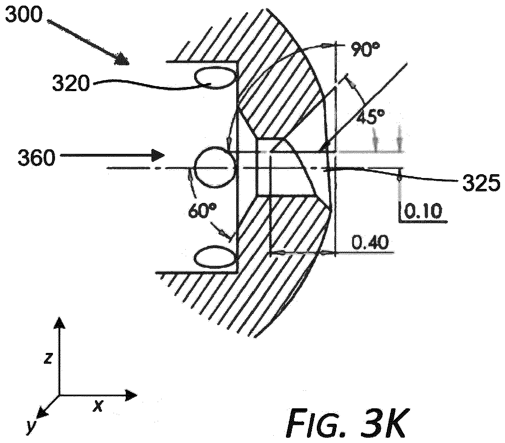

FIG. 3K illustrates a diagram of an example of the catheter tip 300, according to some embodiments of the present disclosure. FIG. 3K depicts an enlarged view of the distal end of the catheter tip 300 referred to as G in FIG. 3J. Shown in FIG 3K is the axial channel 360, the irrigation ports 320, and the distal port 325.

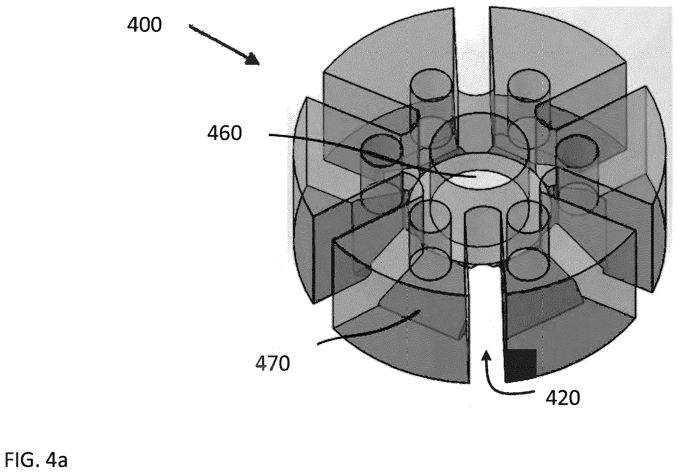

FIG. 4a-d illustrate examples of reflector element 400, according to some embodiments of the present disclosure. In some embodiments, reflector 400 can include a plurality of light reflective facets 470, a plurality of irrigation channels 420, and the axial channel 360. In a preferred embodiment, reflector element 400 includes a cylindrical block 465 of a biocompatible transparent material and includes receptacles 475 for receiving the distal ends of optical fibers that extend through the shaft of the catheter from the proximal end. Light reflective facets 470 preferably are polished, attached or affixed to an indentation in the distal face of reflector element 400.

In some embodiments, reflector element 400 can be made of a biocompatible metal (e.g., stainless steel, titanium alloys, nickel alloys, platinum, platinum alloys, etc.), glass, a biocompatible polymer, or a biocompatible ceramic. In some embodiments, reflector element 400 can be treated after initial manufacture to achieve a smooth surface finish and remove sharp edges, mold flash, or any other potentially harmful aberration, independent of the material used. FIG. 8, described in further detail below, illustrates an example placement and orientation of reflector element 400 disposed in a catheter tip 800.

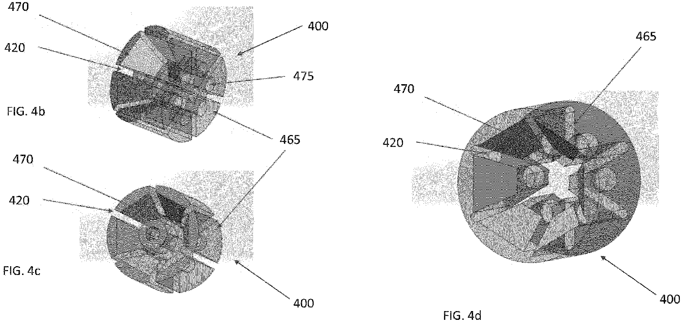

In some embodiments, the irrigation channels 420 that extend longitudinally within and about reflector element 400 can provide enhanced irrigation to an area of the subject being ablated, the catheter tip, and/or the ablation electrode itself. For example, irrigation channels 420 affords the distal end of the catheter tip 300 the ability to provide irrigation fluid proximal to the distal end of the catheter tip 300, which has the additional benefit of cooling the distal end of the catheter tip. By contrast, areflector element lacking one or more irrigation channels 420 may limit irrigation delivery to a tip electrode. Thus, by channeling between facets 470, reflector element 400 shown in the example of FIG. 4 can provide enhanced irrigation to a tip of the ablation catheter, the ablation electrode, and/or an ablation region. Additionally, the channels between facets 470 allow the irrigation fluid to also cool reflector element 400 itself. This cooling of the reflector element 400 may be particularly useful when laser ablation is used, where the laser light used for ablation may be reflected off facets 470 such that the high energy needed for ablation heats reflector element 400.

In some embodiments, mirror or light reflective facets 470 can be polished directly onto reflector element 400. For example, the light reflective facets 370 can be machined or deposited, e.g., by chemical vapor deposition, onto reflector element to create a reflective surface. In this way, reflector element 400 includes a set of integrated facets off which optical interrogation light can reflect, the facets being separated by irrigation channels that pass irrigation fluid through to the tip of the catheter so as to improve cooling at the distal end of the catheter tip. In other embodiments, standalone reflectors can be mounted to the angled facets of reflector element 400, for example, by attaching (e.g., gluing) previously manufactured mirrors to the facets 470. In this way, the facets of reflector element 400 do not have to be so precisely polished, which may aid manufacturing.

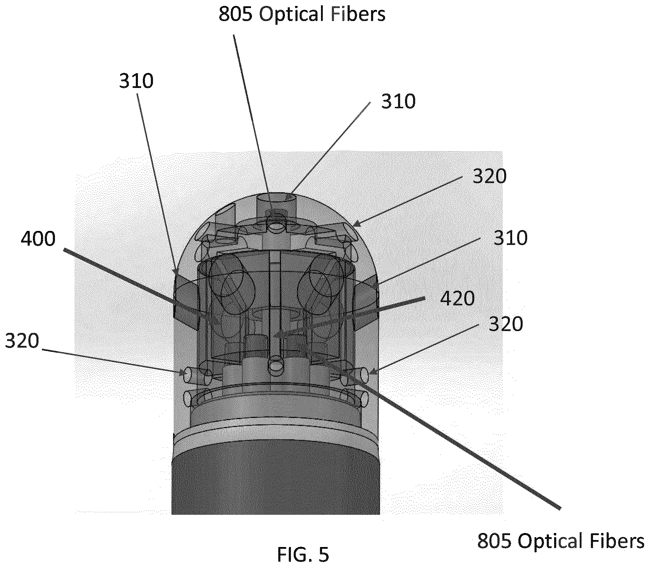

FIG. 5 illustrates a catheter tip assembly 800 incorporating reflector element 400 of FIG. 4. Shown in FIG. 5 are a plurality of optical fibers 805, a plurality of optical ports 810, a plurality of irrigation ports 820, and reflector element 400. Reflector element 400 includes an axial channel 860, a plurality of reflective facets 870 formed on its distal face, and a plurality of irrigation channels 420. As shown, when reflector element 400 is disposed in the interior of the distal tip, the external circumference of the exterior of reflector is in close proximity to the optical ports 810. Each optical fiber 805 is aligned with and disposed within a respective receptacle 475 aligned with a reflective facet 870 on reflector element 400.

In some embodiments, optical interrogation light (e.g., a beam of radiation) can propagate through any, a combination, or all of the optical fibers 805 and transmit to respective reflective facets 870. Light emanating from each optical fiber 805 is centered along a light path on a respective reflective facet 870. Each reflective facet 870, disposed on reflector element 400 and separated from other reflective facets 870 by the irrigation channels 420, reflects a respective beam of radiation toward a respective optical port 810 and onto the tissue outside the optical port. The light is then reflected off the tissue as an optical signal carrying information about the properties of the tissue being interrogated. The optical signal travels back along the path through the optical port to the reflective facet, which then directs the optical signal back into the respective optical fiber 805. The optical signal may then be transmitted to a detector at the proximal end of the catheter, where the optical signal is evaluated.

The light reflective facets 470 divert the light from a direction axial to the optical fiber to a desired angle emitting radially or semi-radially from the catheter tip 300. In some embodiments, the reflector element member may minimize and/or eliminate any need to bend the optical fiber in the catheter tip while illuminating the area radially surrounding the catheter tip. In some embodiments, this concept may be reproduced to achieve any shape and/or orientation along the catheter tip.

In some embodiments, light may also be passed through axial channel 360 of the reflector element 400 to an axially-aligned optical port at the distal end of the catheter tip.

Additionally, in some embodiments, irrigation fluid may flow through the reflector element 400 to reach and cool the distal end of the ablation catheter tip in addition to the proximal end of the ablation catheter tip. FIG. 9 illustrates example irrigation fluid flow paths through an ablation catheter tip 300 containing reflector element 400 therein. As described previously, flowing irrigation fluid through reflector element 400 via the irrigation channels 420 provides thermal control over the distal end of catheter tip assembly 800. As shown in the example of FIG. 9, a distal portion 900 of the catheter tip 300 can provide several paths through which irrigation fluid may flow. As illustrated by the white arrows, a portion of the irrigation fluid may exit the catheter tip through one or more proximal irrigation holes 320, so as to cool and/or wash the proximal end of distal portion 900. Another portion of the irrigation fluid may pass through irrigation channels 420 in reflector element 400 towards the distal end of distal portion 900, and exit the catheter tip through one or more distal irrigation holes 920. This allows the distal end of distal portion 900, including an electrode, electrode housing, or tissue located at the distal end, to be cooled and/or washed by the irrigation fluid. In some embodiments, irrigation fluid can pass from the proximal end of the catheter tip through the central axial channel 360 of reflector element 400 to the distal end of the catheter tip.

In some embodiments, distal portion 900 of catheter tip 300 can deliver illumination light to the area surrounding catheter tip 900 while also irrigating the area surrounding catheter tip 900. While the irrigation fluid passes through channels 420, light may be reflected along the optical path by reflective facets 870. For example, electromagnetic energy in the form of a beam of radiation emitted and/or reflected from the ablation site can be relayed back to the system 100. The light relayed back to the system can be used to observe the ablation process, record information about the ablation process, provide realtime information about the ablation process, and/or observe the irrigation process.

In some embodiments, transmitting the electromagnetic energy through at least one optical fiber can be performed by transmitting the electromagnetic energy through a plurality of optical fibers. For example, as described previously the EM energy can be individually activated and transmitted through any one optical fiber, any portion of the plurality of optical fibers, any combination of optical fibers about the catheter tip 300, or all of the optical fibers as desired, thus providing a controllable optical interrogation area.

In some embodiments, reflector element 400 can have individual mirrors or other reflective surfaces disposed on facets that are not themselves reflective.

In some embodiments, the separation between the reflective facets 470 can prevent undesired cross-talk between individual beams of radiation. As described previously, the beams of radiation reflected by the facets can carry optical interrogation information back from the ablation site and its surrounding area. In some embodiments, separation of the reflective facets 470 provides the ability to harvest information from individual sites that is not skewed with information from neighboring ablation sites. As such, having a non-reflective surface separating the reflective facets 470 provides an improved confidence in information harvested from the ablation site.

In some embodiments, the catheter tip may include passive and fixed optical components (e.g., multiple optical fibers and/or optical fiber bundles), without any mechanical switching or scanning devices in the catheter tip itself. In some embodiments, the plurality of optical ports or view ports in the catheter may have various orientations in the catheter tip, in which each output beam directed from each view port in the catheter may face a different direction. For example, one output beam may be directed forward exiting the catheter tip 300 from the axial channel 360. An additional six beams of radiation may be directed at angles ranging from about 10° to about 170° with respect to the axis of the catheter shaft 106 and catheter tip 300. In some embodiments, there may be any number of beams, view ports, and/or orientations of the view ports in the catheter tip.

In some embodiments, individual beam sources can be aligned with the individual optical fibers and/or optical fiber bundles. Accordingly, referring back to FIG. 3, in some embodiments each of the individual beam sources can be controlled individually or in concert to provide interrogation energy (or, in the case of laser ablation, ablation energy) to a controllable area. In some embodiments, having the reflective facets 370 separated by a non-reflective surface enables an improved and precise controllability during operation.

While embodiments of the reflector element have been described herein with respect to an ablation catheter, a skilled artisan will recognize that a reflector element according the embodiment described herein may be used in other types of catheters or tubular medical devices such as endoscopes.

It is to be appreciated that the Detailed Description section, and not the Summary and Abstract sections, is intended to be used to interpret the claims. The Summary and Abstract sections may set forth one or more but not all exemplary embodiments of the present disclosure as contemplated by the inventor(s), and thus, are not intended to limit the present disclosure and the appended claims in any way.

Embodiments of the present disclosure have been described above with the aid of functional building blocks illustrating the implementation of specified functions and relationships thereof. The boundaries of these functional building blocks have been arbitrarily defined herein for the convenience of the description. Alternate boundaries can be defined so long as the specified functions and relationships thereof are appropriately performed.

The foregoing description of the specific embodiments will so fully reveal the general nature of the disclosure that others can, by applying knowledge within the skill of the art, readily modify and/or adapt for various applications such specific embodiments, without undue experimentation, without departing from the general concept of the present disclosure. Therefore, such adaptations and modifications are intended to be within the meaning and range of equivalents of the disclosed embodiments, based on the teaching and guidance presented herein. It is to be understood that the phraseology or terminology herein is for the purpose of description and not of limitation, such that the terminology or phraseology of the present specification is to be interpreted by the skilled artisan in light of the teachings and guidance.

The breadth and scope of the present disclosure should not be limited by any of the above-described exemplary embodiments, but should be defined only in accordance with the following claims and their equivalents.

## Claims

1. A catheter tip, comprising:
a proximal end configured to attach to a catheter shaft; and
a distal end comprising a housing have an interior, one or more irrigation ports disposed in the housing; and a plurality of optical ports disposed in the housing; and
a reflector element comprising:
a body comprising an optically transparent material;
one or more irrigation channels that extend longitudinally through the body, the the one or more irrigation channels configured to communicate with the one or more irrigation ports; and
a reflective surface disposed on a distal face of the body, the reflective surface configured to direct light along a pathway between an axial direction and a radial direction through at least a subset of the plurality of optical ports, wherein other than the irrigation channels, the body substantially fills the interior of the housing.

2. The catheter tip of claim 1, the proximal end further comprises a plurality of alignment orifices sized and shaped to receive and axially align a corresponding plurality of optical fibers.

3. The catheter tip of claim 1, wherein the body of the reflector element further comprises a plurality of receptacles configured to receive a terminal end of a respective optical fiber.

4. The catheter tip of any of the preceding claims, wherein the reflective surface of the reflector element includes a plurality of reflective facets disposed around an axially aligned opening, the reflective facets separated from one another by a respective one of the one of more irrigation channels.

5. The catheter tip of claim 4, wherein each alignment orifice aligns an optical fiber in the plurality of optical fibers with a respective reflective facet in the plurality of reflective facets.

6. The catheter tip of claim 4, wherein the surfaces of the one or more irrigation channels are non-reflective.

7. The catheter tip of any of the preceding claims, wherein the body comprises a central axial channel, the reflective surface being disposed around the central axial channel.

8. An ablation catheter system, comprising:
a proximal section;
a distal section comprising the catheter tip of any of the preceding claims;
a shaft coupled between the proximal section and the distal section, and
a plurality of optical fibers extending through the shaft from the proximal section to the distal section.

9. The ablation catheter system of claim 8, further comprising a control system.

10. The ablation catheter system of claim 9, wherein the control system is configured to:
transmit optical interrogation light from the proximal section of the catheter through the plurality of optical fibers to the catheter tip; and
analyze an optical signal transmitted through the plurality of fibers from the catheter tip to the proximal section of the catheter.

11. The ablation catheter system of claim 9 or 10, wherein the control system is further configured to individually control the optical interrogation light transmitted through each optical fiber in the plurality of optical fibers.

12. The ablation catheter system of any of claims 8-11, further comprising at least one irrigation channel extending through the shaft and to the distal section of the catheter.
